# EUROPÄISCHE PATENTSCHRIFT

(11) **EP 1 011 753 B1**
(45) Veröffentlichungstag und Bekanntmachung des Hinweises auf die Patenterteilung: **20.07.2005**
(21) Anmeldenummer: 98944887.3
(22) Anmeldetag: 17.09.1998
(51) Int. Cl.: A61M 1/36, A61M 1/10, A61B 5/029

(54) **EINRICHTUNG ZUR UNTERSTÜTZUNG DER LEISTUNG EINES HERZENS**
HEART ASSISTANCE DEVICE
DISPOSITIF D'ASSISTANCE DE L'EFFORT CARDIAQUE

(30) Priorität: 22.09.1997 AT 160097
(43) Veröffentlichungstag der Anmeldung: 28.06.2000
(73) Patentinhaber: Mohl, Werner, 2571 Altenmarkt/Thennenberg (AT)
(72) Erfinder: Mohl, Werner, 2571 Altenmarkt/Thennenberg (AT)
(74) Vertreter: Haffner, Thomas M.
(86) Internationale Anmeldenummer: PCT/AT1998/000225
(87) Internationale Veröffentlichungsnummer: WO 1999/015213

(56) Entgegenhaltungen:
- EP-A- 0 075 606
- EP-A- 0 503 839
- FR-A- 1 187 249
- US-A- 4 255 821
- US-A- 4 674 518
- US-A- 4 778 445
- US-A- 4 804 358
- LEFEMINE ET AL.: "Simplified Bypass for Cardiogenic Shock" A.S.A.I.O TRANSACTIONS, Bd. 33, Nr. 3, September 1987, Seiten 169-176, XP002087620 HAGERSTOWN,MD,USA

## Beschreibung

Die Erfindung bezieht sich auf eine Einrichtung zur Unterstützung der Leistung eines Herzens, bei welcher aus Blutgefäßen Fluid über einen Absaugkatheter und eine Absaugeinrichtung entnommen wird und über einen Rückführungskatheter in Blutgefäße zurückgeführt wird, wobei die Fluidmenge in Abhängigkeit von Meßwerten geregelt ist und wenigstens ein Sensor vorgesehen ist, welcher eine Volumsmessung von Fluid in der Zeiteinheit gestattet und mit einer Auswerteschaltung verbunden ist.

Nach einem Herzversagen, beispielsweise einem Herzinfarkt oder anderen Gründen für die Abnahme der Leistung eines Herzens ist es für die Intensivmedizin von wesentlicher Bedeutung, die Herzfunktion möglichst rasch wiederum zu normalisieren und zu stabilisieren. Insbesondere gilt es dann, wenn beispielsweise die Volumsleistung des Herzens in Folge eines Versagens deutlich reduziert ist, eine entsprechende Peripheriedurchblutung sicher und rasch wiederherzustellen, um sekundäre Schädigungen hintanzuhalten. Die Verwendung von Herz-Lungenmaschinen erlaubt prinzipiell die Aufrechterhaltung wesentlicher Lebensfunktionen. Eine spezielle Anpassung an die jeweiligen konkreten Bedürfnisse erfolgt jedoch mit derartigen Einrichtungen in aller Regel nicht. Vielmehr handelt es sich bei konventionellen Herz-Lungenmaschinen um Einrichtungen, welche unter Verwendung von externen Pumpen eine zwangszirkulation von Blut aufrechterhalten ohne gezielt auf die jeweiligen Bedürfnisse des geschwächten bzw. einem Versagen unterworfenen Herzen einzugehen.

Bei chirurgischen Eingriffen, insbesondere im Bereich der Venen, wurde bereits vorgeschlagen, venendruckgesteuerte Retroinfusion aus bzw. in Körpervenen unter Absaugung von Fluid und Rückführung des Fluids über eine Pumpe vorzunehmen. Zum Einsatz gelangen hiebei konventionelle Katheter, deren Lumen eine Absaugung von Fluid erlauben und über deren Lumen an geeigneter Stelle die Rückführung ermöglicht ist. Bekannte Einrichtungen, insbesondere für die Retroinfusion von Blut in Koronarvenen im Bereich der Myokardprotektion während eines kurzfristigen Koronararterienverschlusses im Rahmen eines kardiologischen Eingriffes, werden zumeist so vorgenommen, daß eine Ballondilatation einer arteriosklerotisch verengten Koronararterie vorgenommen wird. In diesen Fällen kann eine an den jeweils kurzzeitig erfolgenden Eingriff angepaßte Kompensation durch Rückführung von abgesaugtem Blut in Venen vorgenommen werden. Für eine kontinuierliche Restitution der vollen Funktion eines Herzens werden aber diejenigen Kriterien nicht berücksichtigt, welche für die volle Funktion des Herzens relevant wären und es ist daher eine Intensiwersorgung über einen bestimmten zeitraum mit derartigen Einrichtungen nicht vorgesehen. Gleichzeitig muß auch die Versorgung der übrigen Organe aufrechterhalten werden.

Die FR-A-1187249 beschreibt eine Einrichtung zur Unterstützung der Herzleistung, bei welcher aus Blutgefäßen Fluid über einen Absaugkatheter und eine Absaugeinrichtung entnommen wird und über einen Rückführungskatheter in Blutgefäße zurückgeführt wird, wobei die Fluidmenge in Abhängigkeit von Meßwerten geregelt ist. Ferner ist wenigstens ein Sensor mit Auswerteschaltung vorgesehen und ein Signal wird generiert, dessen Abweichung von einem Sollwert zur Regelung der Absaugvorrichtung verwendet wird.

Aus der US-A-4255821 ist im Zusammenhang mit einer Einrichtung zur Unterstützung der Leistung eines Herzens ein Sensor vorgesehen, welcher eine Volumsmenge von Fluid in der Zeiteinheit gestattet und mit einer Auswerteschaltung versehen ist.

Die Erfindung zielt nun darauf ab, eine modulare Einrichtung zu schaffen, mit welcher den jeweiligen Bedürfnissen aller Organsysteme während der Intensivbehandlung eines geschädigten Herzens mit Hilfe von konventionellen und erprobten Bauteilen Rechnung getragen werden kann. Zur Lösung dieser Aufgabe besteht die erfindungsgemäße Einrichtung im wesentlichen darin, daß in der Auswerteschaltung das Verhältnis des diastolischen Volumens zum systolischen Volumen je Herzschlag oder in der Zeiteinheit, insbesondere die Auswurfrate, ausgewertet wird und ein Signal generiert wird und die Absaugeinrichtung in Abhängigkeit von dem generierten Signal geregelt wird. Katheter für die intraventrikulare Erfassung des Kammervolumens sind bekannt. Üblicherweise wird mit derartigen Kathetern, welche in die Herzkammer eingeführt werden, an einer Mehrzahl von Stellen ein elektrisches Feld erzeugt und die Konduktivität gemessen. Aus den Meßwerten kann auf das Kammervolumen geschlossen werden. Dadurch, daß nun eine derartige Volumsmessung dahingehend ausgewertet wird, daß die Leistung des Herzens überprüft wird und insbesondere die sogenannte Auswurfrate bestimmt wird, wird es möglich, eine Optimierung dahingehend zu erreichen, daß ein Signal generiert wird, mit welchem eine Absaugeinrichtung so gesteuert werden kann, daß sie tatsächlich jeweils die erforderliche zusätzliche Leistung erbringt und gleichzeitig das bei jedem Herzschlag in der Herzkammer verbleibende Volumen entsprechend verkleinert. Insgesamt wird somit mit der erfindungsgemäßen Einrichtung selektiv auf eine Verbesserung der Auswurfrate und damit auf eine Verbesserung der Herzleistung abgestellt, wobei die Verwendung von Meßwerten für das diastolische bzw, systolische Volumen hier zu einer selektiven Anpassung an die jeweils optimale erreichbare Reduzierung des Herzinnenraumvolumens darstellt und für die geforderte Herzleistung herangezogen werden kann. Die Absaugeinrichtung soll somit prinzipiell genau diejenige Leistung substituieren, welche vom geschädigten Herzen anfänglich noch nicht erbracht werden kann, worauf weitere Maßnahmen die Herzleistung entsprechend an das normale Maß heranführen können. In vorteilhafter Weise ist der Absaugkatheter mit wenigstens einem Sensor für die Volumsmessung ausgestattet, wobei vorzugsweise der Absaugkatheter als Herzventrikelkatheter ausgebildet ist. In besonders einfacher Weise kann die Absaugeinrichtung als regelbare externe Pumpe ausgebildet sein.

Mit Vorteil ist die erfindungsgemäße Ausbildung hiebei so getroffen, daß die Regelung der externen Absaugeinrichtung so geschaltet ist, daß die Förderleistung der externen Absaugeinrichtung bei geringer Auswurfrate und bei geringer Förderleistung des Herzes erhöht und bei entsprechend höheren Meßwerten erniedrigt wird. Die Förderleistung der Absaugeinrichtung bzw. Pumpe wird somit primär so gesteuert, daß insgesamt eine einem gesunden Herzen entsprechende Auswurfrate simuliert wird. Die Absaugeinrichtung kann hiebei insbesondere getaktet betrieben werden, wodurch in den Blutgefäßen auch die entsprechenden Pulssignale entstehen, welche zu einer Normalisierung der Herzschlagfrequenz und der Leistung des Herzens beitragen. Mit Vorteil wird die Ausbildung hiebei so getroffen, daß die Förderleistung der externen Absaugeinrichtung bzw. Pumpe vorrangig in Abhängigkeit von Meßwerten für die Auswurfrate bis zur Erzielung einer Auswurfrate zwischen 65 und 80 % geregelt wird.

Prinzipiell kann, wie eingangs erwähnt, die Absaugeinrichtung bevorzugt als diskontinuierlich getaktete Pumpe geregelt sein, wodurch auch eine bessere Anpassung an die gewünschte Herzschlagfrequenz erzielt wird. Insbesondere kann es hier vorteilhaft sein, den Takt der externen Absaugeinrichtung fest einzustellen. Im Falle von Tachykardie kann eine derartige fest vorgegebene Taktfrequenz der externen Absaugeinrichtung allerdings zu unerwünschten Interferenzen mit der Herzfrequenz führen, sodaß mit Vorteil hier so vorgegangen wird, daß die Absaugeinrichtung mit dem Herzschlag synchronisiert ist, wobei im Falle von Tachykardie der Takt der Absaugeinrichtung gegenüber dem Herzschlag in einem definierten Verhältnis reduziert ist.

Um die entsprechend getaktete Rückführung des Fluids zu gewährleisten, kann neben der Maßnahme, die externe Absaugeinrichtung diskontinuierlich zu betreiben zusätzlich oder alternativ auch eine getaktete Perfusion mit einem getaktet okkludierenden Katheter vorgenommen werden. Mit Vorteil wird hiebei die erfindungsgemäße Einrichtung so betrieben, daß die Rückführung des Fluids durch Einstellung des Taktes der Absaugeinrichtung der diskontinuierlich fördernden Absaugeinrichtung oder durch getaktete Perfusion mit einem getaktet okkludierenden Katheter während der Diastole erfolgt, wobei im letzten Fall ggf. eine kontinuierlich fördernde externe Absaugeinrichtung bzw. Pumpe eingesetzt ist.

Die Rückführung des Blutes erfolgt bevorzugt wiederum in ein arterielles Gefäß und an geeigneter Stelle nahe der Stelle, an welcher das Blut abgezogen wurde. Auf diese weise wird sichergestellt, daß eine möglichst gleichmäßige Durchblutung aller Gefäße gewährleistet ist.

Eine weitere Verbesserung unter Vewendung des modular aufgebauten Systemes ergibt sich, wenn so vorgegangen wird, daß Blut aus dem rechten Herzen oder großen Venen volumetrisch entnommen wird, in einem Oxygenerator mit O₂ angereichert wird und im Kreislauf gepumpt wird.

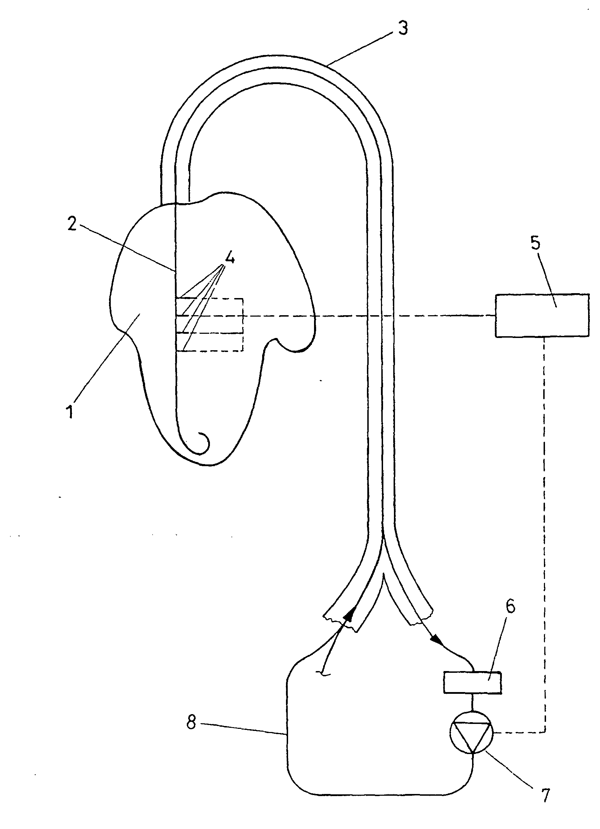

Die erfindungsgemäße Einrichtung wird schematisch anhand der Zeichnung näher erläutert. In dieser ist mit 1 ein Herz bezeichnet, in welches ein Herzventrikelkatheter 2 eingeführt ist. Der Katheter wird hiebei beispielsweise über die Femoralarterie und den Aortenbogen 3 in die Herzkammer eingeführt und trägt eine Reihe von Sensoren 4, über welche das Volumen bestimmt werden kann. Die Meßsignale werden einer Steuereinrichtung 5 zugeführt. Fluid aus der Herzkammer wird über einen Speicherbehälter 6 und eine Absaugeinrichtung 7 abgezogen, wobei die Absaugeinrichtung 7 in Abhängigkeit von den in der Steuereinrichtung 5 generierten Steuersignalen getaktet geregelt ist. Über einen Katheter 8 wird das abgezogene Blut wiederum in die andere Leiste der Arterie rückgeführt. Die Rückführung erfolgt hiebei bevorzugt während der Diastole, sodaß insgesamt eine korrekte Ausbildung von augmentierten Druckwellen in den Blutgefäßen gewährleistet wird.

Die volumetrische Messung in der Herzkammer erlaubt Unterschiede zwischen diastolischen und systolischen Volumen sicher zu erfassen und entsprechende Korrektursignale für die Leistung der getakteten Pumpe zur Verfügung zu stellen. In der Steuerschaltung 5 können darüber hinaus entsprechende Festwerte, wie beispielsweise ein definiertes Herzzeitvolumen, vorgegeben werden, welches bei Abweichung des gemessenen Herzzeitvolumens zur Steuerung der Pumpe herangezogen wird.

Die Rückführung über den Katheter 8 kann über einen konventionellen Ballonkatheter erfolgen, welcher entsprechend getaktet okkludiert wird, sodaß tatsächlich die gerichtete Rückführung während der Diastole gewährleistet wird. Die entsprechenden Meßwerte für die Herzfrequenz bzw. für den korrekten Zeitpunkt der Diastole können hiebei aus EKG-Daten gewonnen werden.

Die Rückführung des Blutes erfolgt in die zentrale Aorta.

## Patentansprüche

1. Einrichtung zur Unterstützung der Leistung eines Herzens mit einem Absaugkatheter (2) und einer Absaugeinrichtung (7) zur Entnahme von Fluid aus Blutgefäßen und einem Rückführungskatheter (8) zur Rückführung des Fluids in Blutgefäße, wobei die Fluidmenge in Abhängigkeit von Meßwerten geregelt ist und wenigstens ein Sensor (4) vorgesehen ist, welcher eine Volumensmessung von Fluid in der Zeiteinheit gestattet und mit einer Auswerteschaltung (5) verbunden ist, **dadurch gekennzeichnet, dass** in der Auswerteschaltung (5) das Verhältnis des diastolischen Volumens zum systolischen Volumen je Herzschlag oder in der Zeiteinheit, insbesondere die Auswurfrate, ausgewertet wird und ein Signal generiert wird und die Absaugeinrichtung (7) in Abhängigkeit von dem generierten Signal geregelt wird.

2. Einrichtung nach Anspruch 1, **dadurch gekennzeichnet, daß** der Absaugkatheter mit wenigstens einem Sensor (4) für die Volumsmessung ausgestattet ist.

3. Einrichtung nach Anspruch 1 oder 2, **dadurch gekennzeichnet, daß** der Absaugkatheter als Herzventrikelkatheter (2) ausgebildet ist.

4. Einrichtung nach Anspruch 1, 2 oder 3, **dadurch gekennzeichnet, daß** die Absaugeinrichtung (7) als regelbare externe Pumpe ausgebildet ist.

5. Einrichtung nach einem der Ansprüche 1 bis 4, **dadurch gekennzeichnet, daß** die Regelung der externen Absaugeinrichtung (7) so geschaltet ist, daß die Förderleistung der externen Absaugeinrichtung (7) bei geringer Auswurfrate und bei geringer Förderleistung des Herzes (1) erhöht und bei entsprechend höheren Meßwerten erniedrigt wird.

6. Einrichtung nach einem der Ansprüche 1 bis 5, **dadurch gekennzeichnet, daß** die Förderleistung der externen Absaugeinrichtung (7) vorrangig in Abhängigkeit von Meßwerten für die Auswurfrate bis zur Erzielung einer Auswurfrate zwischen 65 und 80 % geregelt wird.

7. Einrichtung nach einem der Ansprüche 1 bis 6, **dadurch gekennzeichnet, daß** die externe Absaugeinrichtung (7) als diskontinuierlich getaktete Absaugeinrichtung geregelt ist.

8. Einrichtung nach Anspruch 7, **dadurch gekennzeichnet, daß** der Takt der externen Absaugeinrichtung (7) fest eingestellt oder mit dem Herzschlag synchronisiert ist, wobei im Falle von Tachykardie der Takt der Absaugeinrichtung (7) gegenüber dem Herzschlag in einem definierten Verhältnis reduziert ist.

9. Einrichtung nach einem der Ansprüche 1 bis 8, **dadurch gekennzeichnet, daß** die Rückführung des Fluids durch Einstellung des Taktes der Absaugeinrichtung (7) der diskontinuierlich fördernden Absaugeinrichtung oder durch getaktete Perfusion mit einem getaktet okkludierenden Katheter während der Diastole erfolgt, wobei im letzten Fall ggf. eine kontinuierlich fördernde externe Absaugeinrichtung (7) eingesetzt ist.

10. Einrichtung nach einem der Ansprüche 1 bis 9, **dadurch gekennzeichnet, daß** der Rückführungskatheter (8) in einem arteriellen Gefäß angeordnet ist.

11. Einrichtung nach einem der Ansprüche 1 bis 7, **dadurch gekennzeichnet, daß** Blut aus dem rechten Herzen oder großen Venen volumetrisch entnommen wird, in einem Oxygenerator mit O₂ angereichert wird und im Kreislauf gepumpt wird.

## Claims

1. A device for supporting the output of a heart, including a suction catheter (2) and a suction means (7) for taking fluid from blood vessels and a return catheter (8) for returning said fluid into blood vessels, wherein the amount of fluid is controlled as a function of measured values and at least one sensor (4) is provided, which allows a volume measurement of fluid per time unit and is connected with an evaluation circuit (5), **characterized in that** the ratio of the diastolic volume to the systolic volume per heart beat or per time unit and, in particular, the ejection rate is evaluated in the evaluation circuit (5) and a signal is generated, and the suction means (7) is controlled as a function of the generated signal.

2. A device according to claim 1, **characterized in that** the suction catheter is equipped with at least one sensor (4) for said volume measurement.

3. A device according to claim 1 or 2, **characterized in that** the suction catheter is designed as a heart ventricle catheter -(2),

4. A device according to claim 1, 2 or 3, **characterized in that** the suction means (7) is designed as a regulatable external pump.

5. A device according to any one of claims 1 to 4, **characterized in that** the regulation of the external suction means is controlled in a manner that the flow rate of the external suction means (7) is increased at a low ejection rate and at a low flow rate of the heart (1) and is lowered at accordingly higher measured values.

6. A device according to any one of claims 1 to 5, **characterized in that** the flow rate of the external suction means (7) in the first place is controlled as a function of the measured values of the ejection rate until the attainment of an ejection rate ranging between 65 and 80%.

7. A device according to any one of claims 1 to 6, **characterized in that** the external suction means (7) is controlled as a discontinuously clocked suction means.

8. A device according to claim 7, **characterized in that** the clock pulse of the external suction means (7) is fixed or synchronized with the heart beat, the clock pulse of the suction means (7) in the event of tachycardia being reduced by a defined ratio relative to the heart beat.

9. A device according to any one of claims 1 to 8, **characterized in that** the return of the fluid is effected by the adjustment of the suction means clock pulse of the discontinuously conveying suction means (7), or by clocked perfusion with a clockedly occluding catheter during the diastole, with a continuously conveying external suction means (7) being optionally employed in the latter case,

10. A device according to any one of claims 1 to 9, **characterized in that** the return catheter (B) is arranged in an arterial vessel.

11. A device according to any one of claims 1 to 7, **characterized in that** blood is volumetrically taken from the right heart or from large veins, enriched with O₂ in an oxygenerator and recirculated.

## Revendications

1. Dispositif d'assistance à l'effort cardiaque comportant un cathéter d'aspiration (2) et un dispositif d'aspiration (7) pour le prélèvement de fluide à partir des vaisseaux sanguins et un cathéter de retour (8) pour le retour du fluide dans les vaisseaux sanguins, étant entendu que la quantité de fluide est réglée en fonction de valeurs de mesure et qu'il est prévu au moins un capteur (4), permettant une mesure de volume du fluide dans l'unité de temps et qui est relié à un circuit d'évaluation (5),
**caractérisé en ce que** dans le circuit d'évaluation (5), on évalue le rapport entre le volume diastolique et le volume systolique par battement cardiaque ou dans l'unité de temps, en particulier on évalue la cadence d'éjection, un signal est généré et le dispositif d'aspiration (7) est réglé en fonction du signal généré.

2. Dispositif selon la revendication 1,
**caractérisé en ce que** le cathéter d'aspiration est équipé d'au moins un capteur (4) pour la mesure de volume.

3. Dispositif selon la revendication 1 ou 2,
**caractérisé en ce que** le cathéter d'aspiration est configuré comme cathéter de ventricule cardiaque (2).

4. Dispositif selon la revendication 1, 2 ou 3,
**caractérisé en ce que** le dispositif d'aspiration (7) est configuré comme pompe externe réglable.

5. Dispositif selon l'une des revendications 1 à 4,
**caractérisé en ce que** la régulation du dispositif d'aspiration externe (7) est commutée de telle sorte que la capacité d'aspiration du dispositif d'aspiration externe (7) est augmentée lorsque la cadence d'éjection est faible et que la capacité de refoulement du coeur (1) est faible et elle est réduite lorsque les valeurs de mesure sont plus élevées de façon correspondante.

6. Dispositif selon l'une des revendications 1 à 5,
**caractérisé en ce que** la capacité d'aspiration du dispositif d'aspiration externe (7) est régulée prioritairement en fonction des valeurs de mesure pour la cadence d'éjection jusqu'à l'obtention d'une cadence d'éjection entre 65 et 80 %.

7. Dispositif selon l'une des revendications 1 à 6.
**caractérisé en ce que** le dispositif d'aspiration externe (7) est régulé en tant que dispositif d'aspiration rythmé de façon discontinue.

8. Dispositif selon la revendication 7,
**caractérisé en ce que** le rythme du dispositif d'aspiration externe (7) est réglé de façon fixe ou est synchronisé avec les battements cardiaques, étant entendu qu'en cas de tachycardie le rythme du dispositif d'aspiration (7) est réduit par rapport aux battements cardiaques dans un rapport défini.

9. Dispositif selon l'une des revendications 1 à 8,
**caractérisé en ce que** le retour du fluide s'effectue par réglage du rythme du dispositif d'aspiration (7) du dispositif d'aspiration refoulant de façon discontinue ou par perfusion rythmée avec un cathéter à occlusion rythmée pendant la diastole, étant entendu dans le dernier cas qu'un dispositif d'aspiration externe (7) aspirant en continu entre en action.

10. Dispositif selon l'une des revendications 1 à 9,
**caractérisé en ce que** le cathéter de retour (8) est disposé dans un vaisseau artériel.

11. Dispositif selon l'une des revendications 1 à 7,
**caractérisé en ce que** l'on prélève volumétriquement le sang provenant du coeur droit ou des grosses veines, on l'enrichit en O₂ dans un oxygénateur et on le renvoie par pompage dans la circulation sanguine.
